# EUROPEAN PATENT APPLICATION

(11) **EP 1 787 664 A1**
(43) Date of publication of application: **23.05.2007**
(21) Application number: 04772086.7
(22) Date of filing: 24.08.2004
(51) Int. Cl.: A61L 27/00, A61F 2/10

(54) **PROCESS FOR PRODUCING COLLAGEN SPONGE, PROCESS FOR PRODUCING ARTIFICIAL SKIN, ARTIFICIAL SKIN AND CELL TISSUE CULTURE SUBSTRATE**

(71) Applicant: GUNZE LIMITED, Ayabe-shi, Kyoto 623-8511 (JP)
(72) Inventor: TAIRA, Tsuguyoshi, GUNZE LIMITED Res. Laboratory, Kyoto, 6238512 (JP)
(74) Representative: Hart Davis, Jason
(86) International application number: PCT/JP2004/012125
(87) International publication number: WO 2006/021992

(57) **Abstract**

It is an object of the present invention to provide a method for producing a collagen sponge and a method for producing an artificial skin which can give a high yield through simplified steps, no inferior goods being produced in the finally-obtained collagen sponges and artificial skins; and an artificial skin and a substrate for tissue engineering which respectively comprise a collagen sponge produced by the method for producing a collagen sponge.

The present invention is a method for producing a collagen sponge, wherein a diluted collagen solution having a pH adjusted by use of acetic acid is used.

## Description

### TECHNICAL FIELD

The present invention relates to a method for producing a collagen sponge and a method for producing an artificial skin which can give a high yield through simplified steps; and an artificial skin and a substrate for tissue engineering which respectively comprise a collagen sponge produced by the method for producing a collagen sponge.

### BACKGROUND ART

It is known that in the case of transplanting a collagen sponge as an artificial skin into the part of a disease such as a skin burn, the collagen sponge has an effect of providing countless pores (spaces) suitable for the multiplication of fibroblasts by its porous structure and aiding the multiplication of fibroblasts so as to promote the recovery of the diseased part. A product wherein a water permeation adjusting layer comprising a silicone film and the like is laminated on such a collagen sponge is used as an excellent artificial skin since the product can restrain the transpiration of water, the invasion of microorganisms and the like, and others from the diseased part.

For example, Patent Document 1 discloses, as such an artificial skin, an artificial skin wherein a collagen sponge layer having countless microscopic pores is laminated over a water permeation adjusting layer comprising a thin film of a water permeable polymeric substance so as to interpose an intermediate layer formed by a fibrous material of a bioabsorbable polymeric substance therebetween. Moreover, Patent Document 2 discloses, as a such an artificial skin producing method, a collagen sponge drying method of moistening a collagen sponge with a solution of a hydrophilic organic solvent in water, and then freeze-drying the collagen sponge; and Patent Document 3 discloses a collagen sponge producing method of adding a lipophilic organic solvent into a collagen solution, and then homogenizing the solution to generate foams.

A collagen sponge producing method or an artificial skin having a water permeation adjusting layer comprising a thin film of a water permeable polymeric substance and a collagen sponge layer producing method, which has been published, is, for example, a method disclosed in Non-patent Document 1. An outline thereof is shown in Fig. 1 and Fig. 2.

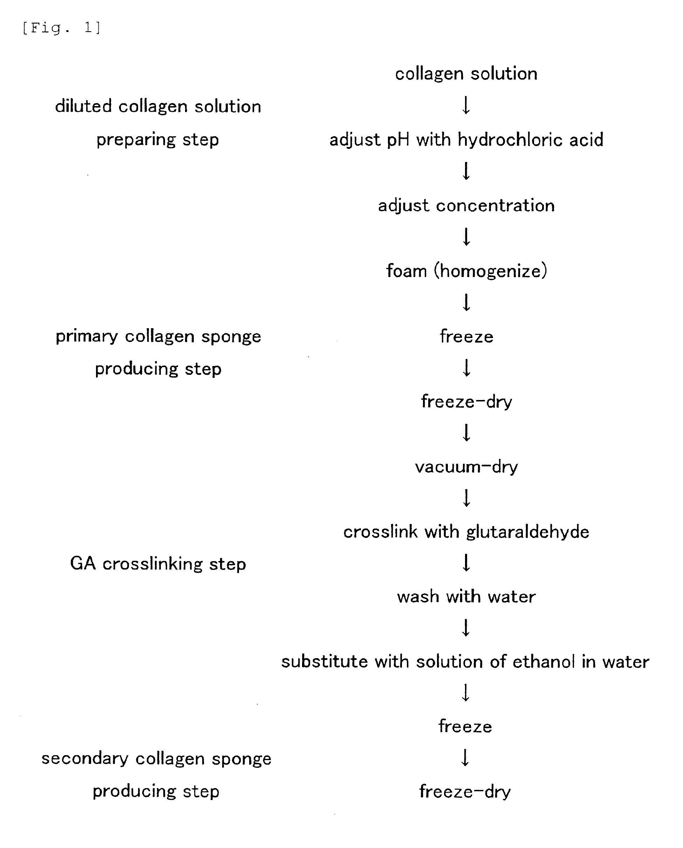

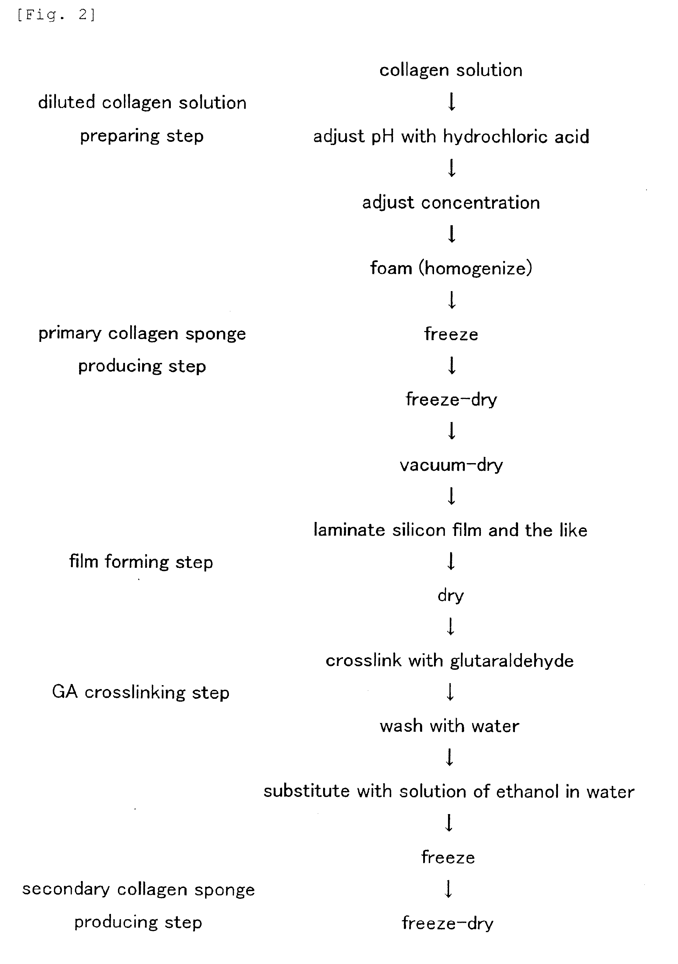

The collagen sponge producing method in Fig. 1 comprises: a diluted collagen solution preparing step of adjusting the pH or the concentration of a collagen solution; a primary collagen sponge producing step of foaming the collagen and then freeze-drying the collagen; a GA crosslinking step of crosslinking the collagen sponge, which has not yet been crosslinked, with glutaraldehyde (GA); and a secondary collagen sponge producing step of freeze-drying the crosslinked collagen sponge.
The artificial skin producing method in Fig. 2 comprises: a diluted collagen solution preparing step of adjusting the pH or the concentration of a collagen solution; a primary collagen sponge producing step of foaming the collagen and then freeze-drying the collagen; a film forming step of laminating a water permeation adjusting layer comprising silicone and the like onto the surface of the collagen sponge, which has not yet been crosslinked; a GA crosslinking step of crosslinking the non-crosslinked collagen sponge with glutaraldehyde (GA); and a secondary collagen sponge producing step of freeze-drying the crosslinked collagen sponge.

However, such conventional collagen sponge producing methods or artificial skin producing methods have problems that the steps thereof are complicated; and inferior goods are produced wherein cracks or cleavages are generated in a finally-obtained collagen sponge or in the surface of a collagen sponge layer of a finally-obtained artificial skin, or a water permeation adjusting layer of the artificial skin is wrinkled, thereby resulting in a fall in the yield.

Patent Document 1: Japanese Patent No. 2987464
Patent Document 2: Japanese Kokoku Publication Hei-7-100
Patent Document 3: Japanese Patent No. 2997823
Non-patent Document 1: Biomaterials 14, 1030 (1993)

### DISCLOSURE OF THE INVENTION

### PROBLEMS WHICH THE INVENTION IS TO SOLVE

In light of the present situation, an object of the present invention is to provide a method for producing a collagen sponge and a method for producing an artificial skin which can give a high yield through simplified steps; and an artificial skin and a substrate for tissue engineering which respectively comprise a collagen sponge produced by the method for producing a collagen sponge.

### MEANS FOR SOLVING THE OBJECT

The present invention is a method for producing a collagen sponge, wherein a diluted collagen solution having a pH adjusted by use of acetic acid is used.

The present invention is a method for producing a collagen sponge, which comprises a step of crosslinking a not-crosslinked collagen sponge in glutaraldehyde-volatilized gas.

The present invention is a method for producing a collagen sponge, which comprises a step of adding glutaraldehyde to a diluted collagen solution, and freeze-drying the resultant to prepare a crosslinked collagen sponge.

The present invention is a method for producing an artificial skin at least comprising a water permeation adjusting layer comprising a thin film of a water permeable polymeric substance, and a collagen sponge layer, wherein a not-crosslinked collagen sponge is subjected to glutaraldehyde crosslinking treatment, the resultant is freeze-dried to prepare a crosslinked collagen sponge, and then the water permeation adjusting layer is laminated onto the crosslinked collagen sponge.
The present invention will be described in detail hereinafter.

In a method for producing a collagen sponge of the present invention, a diluted collagen solution having a pH adjusted by use of acetic acid is used (it may be referred to as improvement in the diluted collagen solution preparation method hereinafter).
The collagen which is the raw material of the collagen sponge is preferably a collagen which does not have any antigenicity by enzymatic treatment of its collagen terminals. As such a collagen, raw materials known in the prior art for a collagen sponge can widely be used, and for example, the following can be used: soluble collagens such as acid-soluble collagen, neutral salt soluble collagen, and enzyme-solubilizable collagen; natural or chemically-modified collagen fibers; and regenerated collagen fiber obtained by making a soluble collagen insoluble.

The collagens, which are each the raw material of the collagen sponge, are each used in the form of a diluted collagen solution obtained by adjusting the pH of a solution of the collagen into about 3 by use of an acid so as to solubilize the solution. In conventional collagen sponge producing methods, hydrochloric acid is used for the adjustment of the pH.
In the method for producing a collagen sponge of the present invention related to improvement in the diluted collagen solution preparation method, acetic acid is used to adjust the pH, thereby making it possible to restrain the generation of cracks or cleavages in a finally-obtained collagen sponge to improve the yield. In the case that the use amount of acetic acid becomes too large by use of only acetic acid so that the concentration of acetic acid becomes too high, hydrochloric acid and the like may be used together.

The reason for the above is not clear, but is thought that: when the pH is adjusted with acetic acid, a collagen sponge having uniform pore diameters can be obtained even if the raw material is frozen as it is with the omission of any foaming operation so as to be freeze-dried; thus, the diameters of the pores in the collagen sponge obtained by use of acetic acid are made uniform, thereby improving the mechanical structure to restrain the generation of cracks or cleavage. As described above, in the method for producing a collagen sponge of the present invention related to improvement in the diluted collagen solution preparation method, a collagen sponge having uniform pore diameters can be obtained even if a foaming operation is omitted, and accordingly, the steps can be simplified.

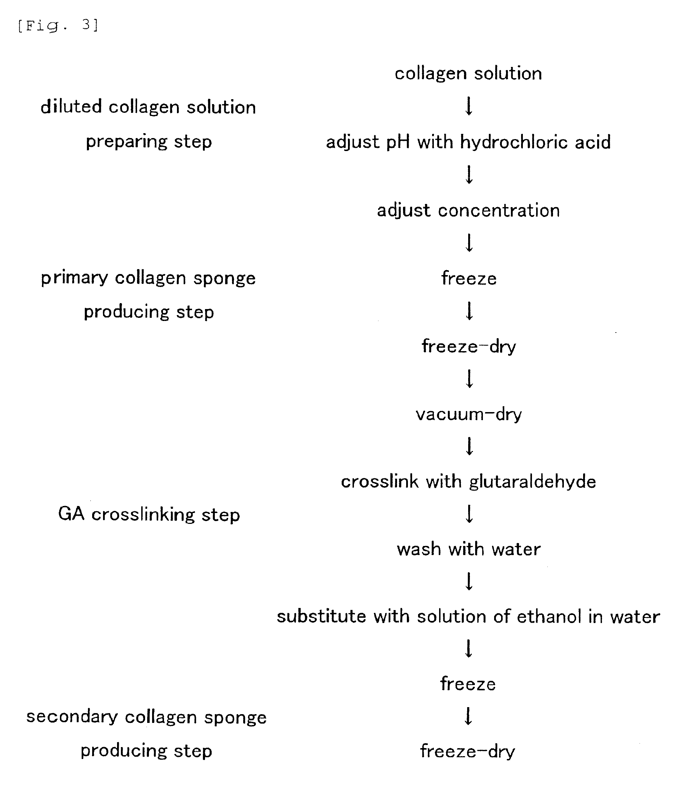

Fig. 3 shows an outline of one example of the method for producing a collagen sponge of the present invention related to improvement in the diluted collagen solution preparation method. In Fig. 3, about others than the characteristic related to the improvement in the diluted collagen solution preparation method, a conventional collagen sponge producing method is described, however, the characteristic may be combined with the different aspects of the method for producing a collagen sponge of the present invention which are described in the present description as the need arises.

A method for producing a collagen sponge of the present invention comprises a step of crosslinking a not-crosslinked collagen sponge in glutaraldehyde-volatilized gas (it may be referred to as improvement in the gas phase crosslinking method hereinafter). In conventional collagen sponge producing methods, a not-crosslinked collagen sponge is immersed into a solution of glutaraldehyde in water, and then the collagen sponge is allowed to stand still for a predetermined time, thereby crosslinking the collagen sponge with GA. In this case, it is necessary to remove remaining glutaraldehyde sufficiently in order to use the resultant collagen sponge clinically since glutaraldehyde generally has high toxicity. However, it is difficult to remove glutaraldehyde completely from the collagen sponge, which has a high hydrophilicity, and conventionally, the collagen sponge has been washed with distilled water and the like many times, and however, the operation is complicated, and further the collagen sponge, which is soft and weak, is damaged at the time of the water washing, and this causes cracks or cleavages in the finally-obtained collagen sponge.

The inventors have paid attention to a matter that glutaraldehyde has volatility, and then found out that when a collagen sponge is crosslinked in glutaraldehyde-volatilized gas, wherein the aldehyde is volatilized, or when a collagen sponge is subjected to vacuum heating treatment, the washing operation for removing remaining glutaraldehyde can be omitted and a fall in the yield based on cracks or cleavages of the collagen sponge can be restrained, and thus, the present invention has been made.

The method for crosslinking a collagen sponge in the glutaraldehyde-volatilized gas is not particularly limited, and may be, for example, a method of putting a solution of glutaraldehyde in water and a not-crosslinked collagen sponge and which does not directly contact the solution of glutaraldehyde in water in an air-tightly closed container, and then allowing the system to stand still for a predetermined time. In this case, the lower limit of the concentration of the solution of glutaraldehyde in water is preferably 1 mM, and the upper limit is preferably 2.5 M. If the lower limit is less than 1 mM, the concentration of glutaraldehyde in the gas phase is low so that the collagen sponge may not be crosslinked or much time may be required for the crosslinking, and if the upper limit is more than 2.5 M, it becomes difficult to handle the solution.

According to the method for producing a collagen sponge of the present invention related to improvement in the gas phase crosslinking method, a collagen sponge in a dry state can be directly crosslinked with glutaraldehyde; therefore, it is unnecessary that the raw material of a collagen sponge is crosslinked with glutaraldehyde in a liquid phase and then the resultant is again freeze-dried to yield a secondary collagen sponge, as performed in conventional collagen sponge producing methods, and as a result, the steps can be largely simplified.

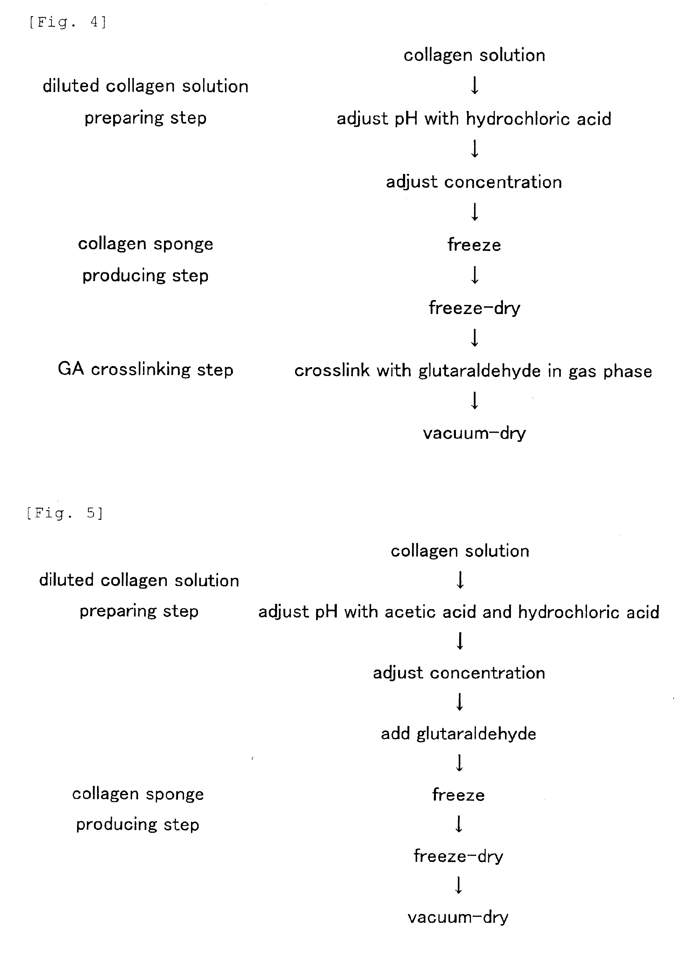

Fig. 4 shows an outline of one example of the method for producing a collagen sponge of the present invention related to improvement in the gas phase crosslinking method. In Fig. 4, about others than the characteristic related to the improvement in the gas phase crosslinking method, a conventional collagen sponge producing method is described, however, the characteristic may be combined with the different aspects of the method for producing a collagen sponge of the present invention which are described in the present description as the need arises.

A method for producing a collagen sponge of the present invention comprises a step of adding glutaraldehyde to a diluted collagen solution, and freeze-drying the resultant to prepare a crosslinked collagen sponge (it may be referred to as improvement in the solution crosslinking method hereinafter).
As shown in Fig. 1, in conventional collagen sponge producing methods, a water permeation adjusting layer is laminated onto a primary collagen sponge, and subsequently the collagen sponge is crosslinked with glutaraldehyde. In the methods, however, it is necessary to use a complicated operation for removing remaining glutaraldehyde, as described above, and moreover, this operation causes a fall in the yield.
The inventors have made eager investigations, and found out that in the case of freezing a diluted collagen solution and vacuum-drying the collagen to produce a collagen sponge, glutaraldehyde is beforehand added into the diluted collagen solution, whereby a crosslinked collagen sponge can be obtained, and thus, the present invention has been made.

Conventionally, it has been considered that: the concentration of collagen in a water solution thereof is low; therefore, the collagen is not crosslinked even if glutaraldehyde is directly added into the diluted collagen solution. However, in the case of freeze-drying a diluted collagen solution into which glutaraldehyde is added, the solution is concentrated in the course of the freeze-drying so that the collagen concentration becomes high, and as a result, the collagen would be crosslinked. Glutaraldehyde has volatility, and in the step of freeze-drying, the gultaraldehyde is vaporized and scattered; thus, glutaraldehyde does not remain in the resultant crosslinked collagen sponge. Moreover, it is unnecessary that a tentatively-produced not-crosslinked collagen sponge is crosslinked with glutaraldehyde in a liquid phase and then the resultant is again freeze-dried to yield a secondary collagen sponge, as performed in conventional collagen sponge producing methods, and as a result, the steps can be largely simplified.

The collagen sponge obtained by the method for producing a collagen sponge of the present invention related to improvement in the solution crosslinking method has better water absorptivity and better cell adhesiveness than collagen sponges produced by conventional methods, reasons for this matter being unclear. Accordingly, in the case of using the collagen sponge obtained by the method for producing a collagen sponge of the present invention related to improvement in the solution crosslinking method, fibroblasts permeate satisfactorily into the collagen sponge, so that the recovery of a disease is promoted, and besides, when this collagen sponge is used as a base for the cell seeding in the regenerative medicine of skin, bones, blood vessels, nerves, and others, the collagen sponge is very useful since the collagen sponge is very good in adhesion of cells thereto.

In the method for producing a collagen sponge of the present invention related to improvement in the solution crosslinking method, the lower limit of the concentration of glutaraldehyde added into the diluted collagen solution is preferably 0.01 mM, and the upper limit is preferably 1 M. If the lower limit is less than 0.01 mM, the collagen sponge is not sufficiently crosslinked, and if the upper limit is more than 1 M, the collagen sponge is crosslinked to an excessively high degree so that the hydrophilicity of the layer of the collagen sponge falls, and as a result, fibroblasts do not permeate satisfactorily into the collagen sponge, so that the recovery of a disease may be delayed.

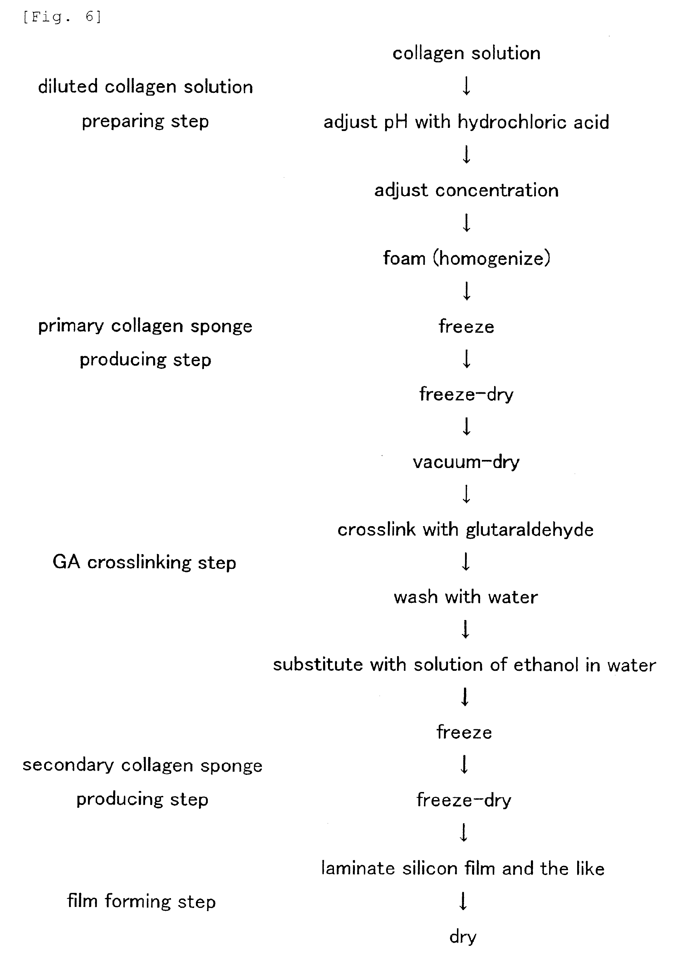

Fig. 5 shows an outline of one example of the method for producing a collagen sponge of the present invention related to improvement in the solution crosslinking method. In Fig. 5, about others than the characteristic related to the improvement in the solution crosslinking method, a conventional collagen sponge producing method is described, however, the characteristic may be combined with the different aspects of the method for producing a collagen sponge of the present invention which are described in the present description as the need arises.

The collagen sponge produced by the method for producing a collagen sponge of the present invention has many microscopic pores, and the lower limit of the pore diameter thereof is preferably 5 µm, and the upper limit is preferably 1000 µm. If the lower limit is less than 5 µm, fibroblasts do not permeate satisfactorily into the collagen sponge, so that the recovery of a disease may be delayed, and if the upper limit is more than 1000 µm, the strength of the collagen sponge may be insufficient or much time is conversely necessary for the recovery of a disease. The lower limit is more preferably 50 µm, and the upper limit is more preferably 130 µm.

The collagen sponge produced by the method for producing a collagen sponge of the present invention can be preferably used as a substrate for tissue engineering which is supplied for the so-called regenerative medicine, the technique in which cells are seeded to construct a tissue.
A substrate for tissue engineering which is obtainable by using the collagen sponge produced by the method for producing a collagen sponge of the present invention is also constitutes the present invention.

The substrate for tissue engineering of the present invention preferably has a reinforcing material to supplement the strength and adjust the shape of a tissue to be regenerated.
The reinforcing material is not particularly limited, and may be, for example, a fibrous material of a bioabsorbable polymeric substance.
Examples of the bioabsorbable polymeric substance include homopolymers or copolymers of glycolic acid, lactic acid, dioxanone, and caprolactone.
The fibrous material may contain, for example, knitting, woven fabric, nonwoven cloth, or any other sheetform fibrous material that is obtained by subjecting thread lines obtained by spinning one or more polymeric substances to treatment for knitting, weaving, knitting and weaving, or production of nonwoven cloth, or that is obtained by making slivers or short fibrous material into a sheet.
The method for the reinforcement based on the reinforcing material is not particularly limited, and for example, the material may be laminated onto a single surface or both surfaces of the collagen sponge, or the reinforcing material may be formed in the collagen sponge when the collagen sponge is produced.

The collagen sponge produced by the method for producing a collagen sponge of the present invention can be used suitably as an artificial skin when a water permeation adjusting layer comprising a thin film of a water permeable polymeric substance is laminated on the collagen sponge.
The artificial skin, which comprises the collagen sponge produced by the method for producing a collagen sponge of the present invention and a water permeation adjusting layer comprising a thin film of a water permeable polymeric substance, also constitutes the present invention.

The water permeation adjusting layer is, for example, a layer comprising a thin film of a water permeable polymeric substance such as silicone, polyurethane, polyacryalte ester, or polymethacrylate ester.
Particularly preferred is silicone since it is excellent in film-formability and biocompatibility.

About the water permeation adjusting layer, the lower limit of the water permeability is preferably 0.1 mg/hr·cm², and the upper limit thereof is preferably 2 mg/hr·cm². If the lower limit is less than 0.1 mg/hr·cm², water remains in the part of a disease so that the recovery thereof may be delayed. If the upper limit is more than 2 mg/hr·cm², water is largely transpired from the part of a disease so that a large burden may be imposed on the patient. The lower limit is more preferably 0.9 mg/hr·cm², and the upper limit is more preferably 1.7 mg/hr·cm².

The thickness of the water permeation adjusting layer is not particularly limited, and is appropriately adjusted into such a thickness that the water permeability can be realized in accordance with the material of the layer. When the layer comprises, for example, silicone, the lower limit thereof is preferably 12.5 µm and the upper limit thereof is preferably 200 µm, and the lower limit thereof is more preferably 25 µm and the upper limit thereof is more preferably 100 µm and the upper limit thereof is further preferably 50 µm.

The artificial skin may have, as a reinforcing material, an intermediate layer comprising a fibrous material of a bioabsorbable polymeric substance on a single surface thereof or between the water permeation adjusting layer and the collagen sponge layer.
Examples of the bioabsorbable polymeric substance include homopolymers or copolymers of glycolic acid, lactic acid, dioxanone, and caprolactone.
The fibrous material may contain, for example, knitting, woven fabric, nonwoven cloth, or any other sheetform fibrous material that is obtained by subjecting thread lines obtained by spinning one or more polymeric substances to treatment for knitting, weaving, knitting and weaving, or production of nonwoven cloth, or that is obtained by making slivers or short fibrous material into a sheet.

The present invention is a method for producing an artificial skin at least comprising a water permeation adjusting layer comprising a thin film of a water permeable polymeric substance, and a collagen sponge layer.
The collagen which is the raw material of the collagen sponge layer is preferably a collagen which does not have any antigenicity by enzymatic treatment of its collagen terminals. As such a collagen, raw materials known in the prior art for a collagen sponge can widely be used, and for example, the following can be used: soluble collagens such as acid-soluble collagen, neutral salt soluble collagen, and enzyme-solubilizable collagen; natural or chemically-modified collagen fibers; and regenerated collagen fiber obtained by making a soluble collagen insoluble.

The collagen sponge layer has many microscopic pores, and the lower limit of the pore diameter thereof is preferably 5 µm, and the upper limit is preferably 1000 µm. If the lower limit is less than 5 µm, fibroblasts do not permeate satisfactorily into the collagen sponge, so that the recovery of a disease may be delayed, and if the upper limit is more than 1000 µm, the strength of the artificial skin may be insufficient or much time is conversely necessary for the recovery of a disease. The lower limit is more preferably 50 µm, and the upper limit is more preferably 130 µm.

The thickness of the collagen sponge layer is preferably 1 mm or more. If the thickness is less than 1 mm, the artificial skin may not be applied to a serious skin burn. The lower limit is more preferably 2 mm, and the upper limit is preferably 4 mm.

The water permeation adjusting layer may be identical with the water permeation adjusting layer which constitutes the artificial skin of the present invention, and the water permeability and the thickness thereof are equivalent with those of the water permeation adjusting layer which constitutes the artificial skin of the present invention.
The artificial skin have, as a reinforcing material, an intermediate layer comprising a fibrous material of a bioabsorbable polymeric substance on a single surface thereof or between the water permeation adjusting layer and the collagen sponge layer.
The bioabsorbable polymeric substance may be equivalent to the bioabsorbable polymeric substance used in the substrate for tissue engineering of the present invention.

Such an artificial skin can be produced by the conventional artificial skin producing method illustrated in Fig. 2; however, the inventors have made eager investigations so as to find out that the producing method is improved to restrain the production of inferior goods based on cracks or cleavages in the collagen sponge, wrinkles of the water permeation adjusting layer, and the like, whereby a high yield can be realized and further the steps can be simplified.

In the method for producing an artificial skin of the present invention, a not-crosslinked collagen sponge is subjected to glutaraldehyde crosslinking treatment, the resultant is freeze-dried to prepare a crosslinked collagen sponge, and then a water permeation adjusting layer is laminated onto the crosslinked collagen sponge (it may be referred to as improvement in the step hereinafter).
As shown in Fig. 2, in conventional artificial skin producing methods, after the step of forming a film, the step of GA crosslinking and freeze-drying are performed to produce a secondary collagen sponge. This is for preventing the collagen sponge, which is soft and weak, from being damaged in the GA crosslinking and washing after the GA crosslinking by forming, in advance, a water permeation adjusting layer having a high strength on a not-crosslinked collagen sponge.
However, the inventors have investigated so as to understand that: through the GA crosslinking, the washing and the freeze-drying step, the collagen sponge shrinks; therefore, when a film is beforehand formed to fix a collagen sponge, the collagen sponge does not resist the shrinkage so that the collagen sponge is cracked or cleaved or the water permeation adjusting layer is wrinkled.

In the method for producing an artificial skin of the present invention related to improvement in the step, a not-crosslinked collagen sponge and is not formed into a film is crosslinked with glutaraldehyde, the resultant is freeze-dried, and then a water permeation adjusting layer is laminated onto the resultant, and this way makes it possible to restrain the generation of cracks or cleaves in the collagen sponge and wrinkles of the water permeation adjusting layer on the basis of the shrinkage of the collagen sponge since the collagen sponge is not fixed when the collagen sponge shrinks.

Fig. 6 shows one example of the method for producing an artificial skin of the present invention related to improvement in the step. In Fig. 6, about others than the characteristic related to the improvement in the step, a conventional artificial skin producing method is described, however, the characteristic may be combined with the different aspects of the method for producing an artificial skin of the present invention which are described in the present description as the need arises.

### EFFECTS OF THE INVENTION

According to the present invention, it is possible to provide a method for producing a collagen sponge and a method for producing an artificial skin which can give a high yield through simplified steps; and an artificial skin and a substrate for tissue engineering which respectively comprise a collagen sponge produced by the method for producing a collagen sponge.

### BEST MODR FOR CARRYING OUT THE INVENTION

The present invention will be described in more detail by way of the following examples; however, the present invention is not limited to only the examples.

### (Example 1)

A collagen solution ("Atelocollagen", manufactured by Nitta Gelatin Inc.) was used as a raw material, and purified water, 5 N acetic acid, and 1 N hydrochloric acid were used to prepare a diluted collagen solution having a concentration of 3 mg/mL and a pH of 3.0.
Into a stainless steel frame (11 cm x 8.5 cm) for freeze-drying was poured 50 g of the resultant diluted collagen solution. The stainless steel frame was cooled to -40°C to freeze the foamed collagen solution. The solution was freeze-dried at 30°C under a vacuum reduced pressure (0.01 mmHg) for 24 hours. Furthermore, the solution was heated and dried at 105°C under a vacuum reduced pressure (0.01 mmHg) for 24 hours to yield a primary collagen sponge.
Next, the collagen sponge was subjected to crosslinking reaction in a 0.2% by weight solution of glutaraldehyde in acetic acid at 5°C for 24 hours. The resultant crosslinked collagen sponge was sufficiently washed with ion exchange water, and then the water was substituted with a 15% solution of ethanol in water.
Thereafter, the resultant was frozen at -110°C, and then freeze-dried at 30°C under a vacuum reduced pressure (0.01 mmHg) for 24 hours to yield a collagen sponge.
A silicone film of 100 µm thickness was caused to adhere onto the resultant collagen sponge, and then the product was dried to yield an artificial skin wherein the silicon film was laminated on a layer of the collagen sponge.

### (Example 2)

A collagen solution ("Atelocollagen", manufactured by Nitta Gelatin Inc.) was used as a raw material, and purified water, 5 N acetic acid, and 1 N hydrochloric acid were used to prepare a diluted collagen solution having a concentration of 3 mg/mL and a pH of 3.0.
To 50 g of the resultant diluted collagen solution was added 0.5 g of chloroform. Into a stainless steel frame (11 cm x 8.5 cm) for freeze-drying was poured the resultant creamy foamed solution. The stainless steel frame was cooled to -40°C to freeze the foamed collagen solution, and the solution was freeze-dried at 30°C under a vacuum reduced pressure (0.01 mmHg) for 24 hours to yield a collagen sponge.
The resultant collagen sponge was put into an air-tight container having a volume of 8,000 mL, and 50 mL of a separately-prepared 2% by weight solution of glutaraldehyde in acetic acid was added thereto in such a manner that the solution was not brought into direct contact with the collagen sponge. This air-tight container was allowed to stand still at 40 °C for 3 hours to crosslink the collagen sponge, and a crosslinked collagen sponge was yielded.
The resultant collagen sponge was heated and dried at 105°C under a vacuum reduced pressure (0.01 mmHg) for 24 hours, and a silicone film of 100 µm thickness was caused to adhere thereon to yield an artificial skin wherein the silicon film was laminated on a layer of the collagen sponge.

### (Example 3)

A collagen solution ("Atelocollagen", manufactured by Nitta Gelatin Inc.) was used as a raw material, and purified water, 5 N acetic acid, and 1 N hydrochloric acid were used to adjust the concentration of collagen into 3 mg/mL and adjust the pH into 3.0, and then glutaraldehyde was further added thereto so as to set the final concentration of glutaraldehyde into 0.05 mM.
Into a stainless steel frame (11 cm x 8.5 cm) for freeze-drying was poured 50 g of the resultant diluted collagen solution containing glutaraldehyde. The stainless steel frame was cooled to -40°C to freeze the foamed collagen solution. The solution was freeze-dried at 30°C under a vacuum reduced pressure (0.01 mmHg) for 24 hours. Furthermore, the solution was heated and dried at 105°C under a vacuum reduced pressure (0.01 mmHg) for 24 hours to yield a crosslinked collagen sponge.
A silicone film of 100 µm thickness was caused to adhere onto the resultant crosslinked collagen sponge, and then the product was dried to yield an artificial skin wherein the silicon film was laminated on a layer of the collagen sponge.

### (Example 4)

A collagen solution ("Atelocollagen", manufactured by Nitta Gelatin Inc.) was used as a raw material, and purified water, and 1 N hydrochloric acid were used to prepare a diluted collagen solution having a concentration of 3 mg/mL and a pH of 3.0.
To 50 g of the resultant diluted collagen solution was added 0.5 g of chloroform, and a homogenizer ("Excel Auto Homogenizer", manufactured by Nippon Seiki Co., Ltd.) was used to homogenize the solution at 6000 rpm for 1 minute. The resultant foamed solution was poured into a stainless steel frame (11 cm x 8.5 cm) for freeze-drying. The stainless steel frame was cooled to -40°C to freeze the foamed collagen solution. The solution was freeze-dried at 30°C under a vacuum reduced pressure (0.01 mmHg) for 24 hours. Furthermore, the solution was heated and dried at 105°C under a vacuum reduced pressure (0.01 mmHg) for 24 hours to yield a primary collagen sponge.
The resultant primary collagen sponge was immersed into a 0.2% by weight solution of glutaraldehyde in acetic acid to conduct crosslinking reaction at 5°C for 24 hours. The resultant crosslinked collagen sponge was sufficiently washed with ion exchange water, and then the water was substituted with a 15% solution of ethanol in water.
Thereafter, the resultant was frozen at -110°C, and then freeze-dried at 30°C under a vacuum reduced pressure (0.01 mmHg) for 24 hours to yield a secondary collagen sponge, and a silicone film of 100 µm thickness was caused to adhere onto this secondary collagen sponge to laminate the silicone film onto a layer of the collagen sponge and the product was dried to yield an artificial skin.

### (Comparative Example 1)

A collagen solution ("Atelocollagen", manufactured by Nitta Gelatin Inc.) was used as a raw material, and purified water, and 1 N hydrochloric acid were used to prepare a diluted collagen solution having a concentration of 3 mg/mL and a pH of 3.0.
To 50 g of the resultant diluted collagen solution was added 0.5 g of chloroform, and a homogenizer ("Excel Auto Homogenizer", manufactured by Nippon Seiki Co., Ltd.) was used to homogenize the solution at 6000 rpm for 1 minute. The resultant creamy foamed solution was poured into a stainless steel frame (11 cm x 8.5 cm) for freeze-drying. The stainless steel frame was cooled to -40°C to freeze the foamed collagen solution, and the solution was freeze-dried at 30°C under a vacuum reduced pressure (0.01 mmHg) for 24 hours. Furthermore, the solution was heated and dried at 105°C under a vacuum reduced pressure (0.01 mmHg) for 24 hours to yield a primary collagen sponge.
A silicone film of 100 µm thickness was caused to adhere onto the resultant primary collagen sponge, and then the product was dried.
Next, the product was subjected to crosslinking reaction in a 0.2% by weight solution of glutaraldehyde in acetic acid at 5°C for 24 hours. The resultant crosslinked collagen sponge was sufficiently washed with ion exchange water, and then the water was substituted with a 15% solution of ethanol in water.
The resultant was frozen at -80°C, and then freeze-dried at 30°C under a vacuum reduced pressure (0.01 mmHg) for 24 hours to yield an artificial skin wherein the silicon film was laminated on a layer of the collagen sponge.

### (Evaluation)

About 100 pieces of the artificial skin produced in each of Examples 1 to 4, and Comparative Example 1, cracks in their collagen sponge layers, peeling of their silicone films, and wrinkles of the silicone films were examined with the naked eye, and inferior pieces were removed, and then the yield was calculated. For the calculation of the yield, any piece wherein cracks in its collagen sponge layer, peeling of its silicone film, and wrinkles of the silicone film were generated together was counted as one piece.

**[Table 1]**

| | Piece(s) wherein the collagen sponge layer was cracked | Piece(s) wherein the silicon layer was peeled | Piece(s) wherein the silicon layer was wrinkled | Yield (%) |
|---|---|---|---|---|
| Example 1 | 5 | 2 | 1 | 85 |
| Example 2 | 1 | 0 | 0 | 93 |
| Example 3 | 1 | 0 | 0 | 97 |
| Example 4 | 6 | 0 | 0 | 85 |
| Comparative Example 1 | 15 | 8 | 3 | 70 |

### INDUSTRIAL APPLICABILITY OF THE INVENTION

According to the present invention, it is possible to provide a method for producing a collagen sponge and a method for producing an artificial skin which can give a high yield through simplified steps; and an artificial skin and a substrate for tissue engineering which respectively comprise a collagen sponge produced by the method for producing a collagen sponge.

### BRIEF DESCRIPTION OF THE DRAWINGS

Fig. 1 is a schematic view showing one example of a conventional collagen sponge producing method.
Fig. 2 is a schematic view showing one example of a conventional artificial skin producing method.
Fig. 3 is a schematic view showing one example of a method for producing a collagen sponge of the present invention related to improvement in the diluted collagen solution preparation method.
Fig. 4 is a schematic view showing one example of a method for producing a collagen sponge of the present invention related to improvement in the gas phase crosslinking method.
Fig. 5 is a schematic view showing one example of a method for producing a collagen sponge of the present invention related to improvement in the solution crosslinking method.
Fig. 6 is a schematic view showing one example of a method for producing an artificial skin of the present invention related to improvement in the step.

## Claims

1. A method for producing a collagen sponge,
wherein a diluted collagen solution having a pH adjusted by use of acetic acid is used.

2. A method for producing a collagen sponge,
which comprises a step of crosslinking a not-crosslinked collagen sponge in glutaraldehyde-volatilized gas.

3. A method for producing a collagen sponge,
which comprises a step of adding glutaraldehyde to a diluted collagen solution, and freeze-drying the resultant to prepare a crosslinked collagen sponge.

4. An artificial skin,
which comprises a collagen sponge produced by the method for producing collagen sponge according to claim 1, 2 or 3, and a water permeation adjusting layer comprising a thin film of a water permeable polymeric substance.

5. A substrate for tissue engineering,
which is obtainable by using the collagen sponge produced by the method for producing a collagen sponge according to claim 1, 2 or 3.

6. The substrate for tissue engineering according to claim 5,
which comprises a reinforcing material.

7. A method for producing an artificial skin at least comprising a water permeation adjusting layer comprising a thin film of a water permeable polymeric substance, and a collagen sponge layer,
wherein a not-crosslinked collagen sponge is subjected to glutaraldehyde crosslinking treatment, the resultant is freeze-dried to prepare a crosslinked collagen sponge, and then the water permeation adjusting layer is laminated onto the crosslinked collagen sponge.
